# EUROPÄISCHE PATENTSCHRIFT

(11) **EP 3 203 231 B1**
(45) Veröffentlichungstag und Bekanntmachung des Hinweises auf die Patenterteilung: **04.07.2018**
(21) Anmeldenummer: 16000276.2
(22) Anmeldetag: 04.02.2016
(51) Int. Cl.: G01N 33/50

(54) **VERFAHREN ZUR VORBEREITUNG DER ANALYTIK VON PROBEN BIOLOGISCHEN URSPRUNGS**
METHOD OF PREPARING THE ANALYSIS OF SAMPLES OF BIOLOGICAL ORIGIN
PROCEDE DE PREPARATION D'ANALYSE D'ECHANTILLONS D'ORIGINE BIOLOGIQUE

(43) Veröffentlichungstag der Anmeldung: 09.08.2017
(73) Patentinhaber: R-Biopharm Aktiengesellschaft, 64297 Darmstadt (DE)
(72) Erfinder: LACORN, Markus, 65375 Oestrich-Winkle (DE); WINKLE, Johannes, 69493 Hirschberg (DE); BLÖDORN, Dirk, 65931 Frankfurt am Main (DE); GARRIDO, Gilbert, 64293 Darmstadt (DE)
(74) Vertreter: Baumbach, Friedrich

(56) Entgegenhaltungen:
- CN-A- 101 054 370
- US-A- 3 540 455
- BUOGO ET AL: "LA determinatzione della vitamin C negli alimenti (latte, limoni e frutta)", ANNALI DI CHIMICA APPLICATA,, Bd. 25, 1. Januar 1935 (1935-01-01), Seiten 679-684, XP009189414, ISSN: 0365-1037
- LANG W: "Untersuchungen über den Zustand der einfachen und hochmolekularen Polyphenole in der pflanzlichen Zelle", PLANTA MEDICA, THIEME VERLAG, DE, Bd. 2, 1. Januar 1956 (1956-01-01), Seiten 33-40, XP009189413, ISSN: 0032-0943
- MARKUS LACORN ET AL: "Decoloration of wine and subsequent enzymatic quantification of histamine", BIO WEB OF CONFERENCES, Bd. 5, 1. Januar 2015 (2015-01-01), Seite 02019, XP055263229, DOI: 10.1051/bioconf/20150502019
- W Tschopp: "59 Über die Ascorbinsäure-Bestimmung 1 iin Harn", , 1. Januar 1936 (1936-01-01), XP055265047, Gefunden im Internet: URL:http://www.degruyter.com/dg/viewarticl e.fullcontentlink:pdfeventlink/$002fj$002f bchm2.1936.244.issue-1-2$002fbchm2.1936.24 4.1-2.59$002fbchm2.1936.244.1-2.59.pdf?for mat=INT&t:ac=j$002fbchm2.1936.244.issue-1- 2$002fbchm2.1936.244.1-2.59$002fbchm2.1936 .244.1-2.59.xml [gefunden am 2016-04-13]
- Thomas A Keil ET AL: "Insects as Model Systems in cell Biology" In: "Electron Microscopy of Model Systems; III. Methods", 1 January 2010 (2010-01-01), Elsevier, XP055424083, ISBN: 978-0-12-381007-6 pages 372-372, * S 372, Abs "Lanthanum" *

## Beschreibung

### Gebiet der Erfindung

Die Erfindung betrifft ein Verfahren zur Entfernung von störenden Bestandteilen, insbesondere von Polyphenolen und Anthocyanen, aus einer zu untersuchenden Probe biologischen Ursprungs. Anwendungsgebiete sind die Lebensmittelindustrie, die Futtermittelindustrie oder die Biotechnologie

### Hintergrund der Erfindung

Die Lebensmittelanalytik befasst sich mit der Untersuchung der Zusammensetzung von Lebensmitteln und deren Veränderung bei Herstellung, Lagerung und Zubereitung. Insbesondere das Aufspüren und Bestimmen von Allergien auslösenden Substanzen spielt dabei eine immer größere Rolle, da Nahrungsmittelallergien und Unverträglichkeiten ein immer größeres Problem darstellen. So leiden beispielsweise Menschen mit Histaminintoleranz nach dem Genuss von Histamin-haltigen Lebensmitteln wie beispielsweise Rotwein an Hautausschlägen, Kopfschmerzen, Durchfall, Fließschnupfen, Herzklopfen, Brechreiz oder vielen anderen Problemen. Histamin-"Allergikern" wird deshalb der Verzehr von Wein, speziell Rotwein nicht empfohlen. Durch bakterielle Proteolyse und anschießendem Umsatz von freigesetztem Histidin kann während der Lagerung von Wein im Fass oder unter ungünstigen Bedingungen im Tank Histamin gebildet werden. Laut Literatur kommt Histamin in Wein über einen Konzentrationsbereich von 0 - 20 mg/L vor. Die höchsten Konzentrationen werden in Rotwein beobachtet aber auch Weiß- und Roseweine weisen regelmäßig geringe Gehalte auf. Wie eingangs erwähnt, besteht ein großes Interesse Lebensmittel auf solche Allergien oder Unverträglichkeiten auslösenden Substanzen hin zu untersuchen. Allerdings erweisen sich die Ausgangsproben oftmals als nicht geeignet für schnelle und preiswerte analytische Methoden, da sie mögliche Störsubstanzen enthalten können, die dann vorab aufwendig entfernt werden müssen.

Andere wichtige Bereiche der Lebensmittelanalytik stellen die Rückstandsuntersuchungen von Antibiotika und Hormonen dar. Für diese Substanzen liegen zum Teil Grenzwerte zum Teil aber auch Nulltoleranz-Bedingungen vor, d. h. die Anwesenheit selbst bei sehr niedrigen Konzentrationen bewirkt in diesen Fällen eine Nicht-Verkehrsfähigkeit des Lebensmittels. Die Messbereiche der verwendeten Methoden betragen häufig ng/kg und sind aus diesem Grund empfindlich gegenüber Störsubstanzen. Als Beispiel sei an dieser Stelle die Bestimmung des künstlichen Hormons Clenbuterol aus der Gruppe der β-Agonist in Futtermitteln, Leber und Urin genannt.

### Stand der Technik

Bisher erfolgte die Bestimmung des Histamingehalts aus Weinproben größtenteils mittels HPLC. HPLC-Analysen zeichnen sich generell durch einen hohen personellen und apparativen Aufwand und bedingt dadurch hohe Kosten aus. Die Präzision ist in der Regel ausreichend, allerdings ist die Wiederfindung in einigen Fällen nicht immer optimal. Grund dafür ist der Einsatz von Derivatisierungsreagenzien, welche in ausreichender Menge der (extrahierten) Probe zugesetzt werden müssen und bei ungenauer Anwendungsdauer bzw. Temperatur zu wenig präzisen Ergebnissen führen können. Aufgrund der genannten Nachteile ist diese Methode nicht Mittel der Wahl, um Lebensmittel wie Weinproben schnell, einfach und kostengünstig zu analysieren.

Eine benutzerfreundliche und deutlich preiswertere alternative Methode wäre die enzymatische Bestimmung des Histamingehalts (z. B. mit Histamin-Dehydrogenase) bzw. die enzym-immunologische Bestimmung (ELISA). Allerdings führten bisherige Versuche zu keinen befriedigenden Ergebnissen. Es wird vermutet, dass Weinbestandteile wie beispielsweise Anthocyane und Polyphenole mit den Substraten bei der Enzymbestimmung bzw. den Antikörpern beim ELISA wechselwirken und demzufolge vor der Analytik entfernt werden müssen.

Im Fall von Clenbuterol werden sehr erfolgreich chromatographische Methoden wie z. B. LC in Kombination mit massenspektrometrischen Detektoren zur Überprüfung eines illegalen Einsatzes eingesetzt. Diese Verfahren sind wie bereits dargelegt sowohl apparativ als auch personell aufwendig und zeichnen sich generell durch hohe Kosten aus. Daher werden oftmals Screeningmethoden wie ELISA vorgeschaltet, um nur verdächtige Proben in den aufwendigen Verfahren testen zu müssen. Diese Screeningmethoden zeigen häufig Matrixeffekte durch matrixspezifische Störsubstanzen (z. B. Polyphenole in Futtermitteln). Als Folge davon können vermehrt falsche-positive Ergebnisse in der Screnningmethode erhalten werden, wodurch die Probenanzahlen für die nachgeschaltete Bestätigungsmethoden zunehmen.

In der Vergangenheit gab es einige Versuche, Polyphenole im Sinne einer Probenvorbereitung mit Hilfe von Schwermetallen zu entfernen. So wird beispielsweise In Annali di Chimica Applicata (1935) 25:679-684 die Verwendung von Hg (II) zur Fällung von Polyphenolen untersucht. US 3540455 A beschreibt die Entfernung von Polyphenolen aus Tabak mit Hilfe von Bleiacetat order Bariumacetat. Planta Med. (1956) 2:33-40 beschreibt die Verwendung von Zn (II) zur Entfernung von Polyphenolen aus Proben pflanzlichen Ursprungs. Allerdings werden aus Umweltschutzgründen Pb und Hg wenn möglich in der modernen Analytik nicht mehr eingesetzt.

CN 101054370, Cheng et al. 2006 (Yingyong Huagong (2006), 35 (4): 259-262) oder Cheng et al. 2007 (Fujian Shifan Daxue Xuebao, Ziran Kexueban (2007), 23 (1): 105-108) beschreiben die Präparation von Tee-Polyphenol-Lanthan-Komplexen. Ziel dieser Dokumente ist es, die gesundheitsfördernde antimikrobielle und antioxidative Wirkung von Polyphenolen aus grünem Tee in den Polyphenol-seltenen Erden-Komplexen zu untersuchen. Die Verwendung von seltenen Erden erfolgt allerdings nicht zur quantitativen Entfernung von Polyphenolen im Sinne einer Probenvorbereitung für eine anschließende Analytik.

Lacorn et al. (BIO Web of Conferences (2015) Bd. 5, Seite 02019) offenbart einen Testkit zur Entfernung von Polyphenolen und Anthocyanen aus Wein mit anschließender Quantifizierung von Histamin, ohne dabei auf die genaue Zusammensetzung der Reagenzien und das Wirkungsprinzip einzugehen.

Tschopp W. (Hoppe-Seyler's Zeitschrift für physiologische Chemie (1936) 244: 59-77) beschreibt die Verwendung von Hg(II)-Acetat zur Entfernung störender reduzierender Substanzen aus Urin. Die Entfernung dieser störenden Substanzen dient der Probenvorbereitung zur Ascorbinsäure-Quantifizierung in Urin, welche durch den Author zusammenfassend als ungeeignet eingestuft wird. In einem Unterkapitel wird die Verwendung von Pb(II)-Acetat genannt, diese aber sofort als ungeeignet dargestellt, weil der Analyt Ascorbinsäure mit gefällt wird.

In Keil et al. (Electr. Micros. of Model Systems; III. Methods, 1(2010): 363-394) werden die Fällungseigenschaften von Lanthan diskutiert. So wird wasserlösliches dreiwertiges Lanthanidsalz von Phosphatpuffer gefällt.

### Ziel und Aufgabe der Erfindung

Die Erfindung hat das Ziel, Proben biologischen Ursprungs für die Analytik - z. B. für enzymatische Analysen, immun-turbimetrische Analysen, Antikörper-basierte Systeme wie z. B. Lateral-Flow Systeme und ELISA, Mikrofluidik oder Chromatographieverfahren derart vorzubereiten, dass eine exakte Analyse gewährleistet ist. Daraus leitet sich die Aufgabe der vorliegenden Erfindung ab, die Analytik von Proben derart zu verbessern, dass die Analytik störende Substanzen im Vorfeld entfernt und damit Fehlerquellen bei der anschließenden Analytik eliminiert werden. Insbesondere leitet sich daraus die Aufgabe ab, störende Polyphenole und/oder Anthocyane aber auch andere strukturell verwandte Substanzen aus Proben biologischen Ursprungs effektiv und kostengünstig zu entfernen.

Diese Aufgabe wird durch ein Verfahren gemäß Anspruch 1 gelöst. Weitere mögliche Ausführungsformen ergeben sich aus den Unteransprüchen, der Beschreibung und den Beispielen.

### Wesen der Erfindung

Überraschend wurde gefunden, dass mit Hilfe von wasserlöslichen Salzen von dreiwertigen Lanthaniden wie z. B. Cer (III) oder Lanthan (III) Störsubstanzen aus Weinproben eliminiert werden können und eine anschließende enzymatische bzw. immunologische Bestimmung der Histamin-Konzentration dadurch direkt durchführbar wird. Nachfolgende Untersuchungen zeigten, dass es sich bei diesen Störsubstanzen um Polyphenole und Anthocyane bzw. strukturell verwandte Substanzen handelt, die durch die mehrwertigen Metallionen gefällt werden.

Das erfindungsgemäße Verfahren ist dadurch gekennzeichnet, dass wasserlösliche Salze von dreiwertigen Lanthaniden zur Fällung von Polyphenolen und/oder Anthocyanen aus biologischen Proben eingesetzt werden.

Es zeigte sich, dass der pH-Wert und die Konzentration des mehrwertigen Kations einen großen Einfluss auf die Fällungsreaktion haben. So werden unter Einsatz von La (III) und 0.325 M Puffer optimale Ergebnisse geliefert, während für Yb (III) nur unter Einsatz von 0.3 M Puffer optimale Ergebnisse erhalten werden.

Andere dreiwertige Metallionen wie z. B. Y (III), Al (III) und In (III) sind zwar in der Lage Rotweine vollständig zu entfärben, zeigen aber beim Einsatz im analytischen System z. B. Enzymatik Wiederfindungsprobleme. Dennoch ist es denkbar durch Anpassung der Konzentrationen an Fällungsreagenzien auch hier zu quantitativen Wiederfindungen zu gelangen.

Darüber hinaus zeigte sich, dass im Anschluss an die Fällungsreaktion unter Verwendung von wasserlöslichen Salzen von dreiwertigen Lanthaniden eine Behandlung des Ansatzes mit geeigneten Puffern wie beispielsweise mit Phosphat-, Sulfat und Oxalatpuffern insofern essentiell ist, als das dadurch überschüssige dreiwertige Lanthanide gefällt werden, ohne in der anschließenden Analytik zu stören.

Der resultierende Überstand wird anschließend für die Analytik wie beispielsweise die enzymatische, enzymimmunologische bzw. flüssigchromatographische Bestimmung des Histamin-Gehaltes oder Clenbuterol-Gehaltes eingesetzt.

Die Erfindung wird nachfolgend an der Bestimmung der Histamin-Konzentration in Weinen näher erläutert. Es wurde eine Extraktionsmethode für alle Weintypen entwickelt, mit der Polyphenole und Anthocyane entfernt werden können und damit die enzymatische Bestimmung der Histamin-Konzentration dieser Weinproben im Anschluss ermöglicht wird.

Ein typischer Ansatz ist die Fällungsreaktion mit Lanthan (III) als wasserlösliches Salz von dreiwertigem Lanthanid:
Nachfolgend ist als Beispiel ein bevorzugter Ansatz für die Aufarbeitung von Wein und die anschließende enzymatische Bestimmung des Histamin-Gehalts aufgeführt.

Es ist aber auch möglich, andere Proben biologischen Ursprungs zu untersuchen wie beispielsweise Futtermittel, Leber, Hefe, Blut oder Urin.

### Beispiel

200 µl Probe (z. B. Rotwein, Rosewein oder Weißwein)
200 µl Tris-Puffer (0,325 M, pH 9,0)
200 µl LaNO₃ (0,1 M)
Vortexen
5 min bei Raumtemperatur inkubieren
Zentrifugieren (14 000 g, 2 min)
500 µl Überstand abnehmen
100 µl Na-Phosphatpuffer (0,5 M, pH 7,0)
Vortexen
5 min bei Raumtemperatur inkubieren
Zentrifugieren (14 000 g, 2 min)

Der abschließende Zentrifugationsschritt zur Abtrennung des Niederschlags kann auch durch Sedimentations- oder Filtrationsmethoden ersetzt werden.

Der Überstand kann nun für die anschließende Analytik wie beispielsweise die enzymatische Bestimmung des Histamin-Gehalts der Ausgangsprobe verwendet werden. Nachfolgend ist ein typischer Ansatz einer solchen enzymatischen Histaminbestimmung unter Verwendung des kommerziellen Testkits RIDASCREEN® Histamine (enzymatic) der Firma R-Biopharm (Katalog Nr. R1605) aufgeführt (weitere Informationen sind unter Beispiel 3 aufgeführt):
150 µl Tris-Puffer (0.325 M, pH 9)
mit 100 µl Probe bzw. Kalibrator versetzen
vorsichtig manuell schütteln
3 min Inkubation bei Raumtemperatur
die Extinktion A1 bei 450 nm messen
10 µl Histamindehydrogenase zugeben
vorsichtig manuell schütteln.
10 min Inkubation bei Raumtemperatur

Die Extinktion A2 bei 450 nm messen.
unter Berücksichtigung des Verdünnungsfaktors die Konzentrationen der Proben anhand der erstellten Standardkurve berechnen.

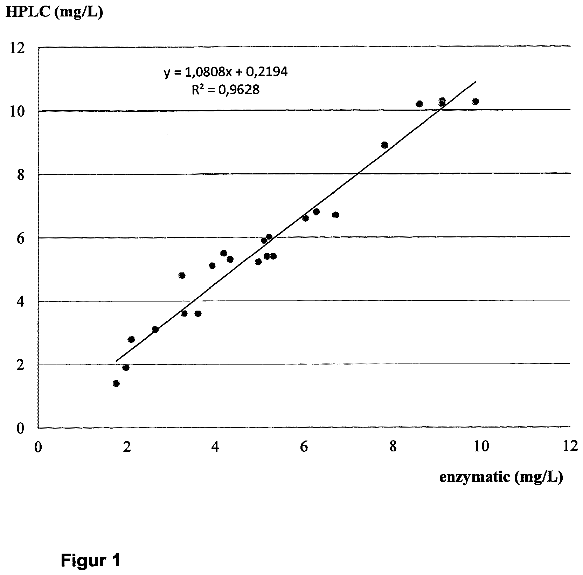

Zur Überprüfung der Effektivität der Fällung von Störsubstanzen mit Hilfe von wasserlöslichen Salzen von dreiwertigen Lanthaniden wurden Vergleichsmessungen zwischen HPLC und Enzymatik unternommen. Untersucht wurden 23 verschiedene Rotweine. Die Ergebnisse in Figur 1 verdeutlichen die sehr gute Vergleichbarkeit der beiden Methoden. Die dazu dazugehörigen Rohdaten sind in folgender Tabelle 1 dargestellt.

**Tabelle 1: Vergleichsuntersuchungen HPLC vs. Enzymatik**

| Weinprobe | Histamin-Gehalt nach Fällung der Polyphenole und Anthocyane (enzymatisch bestimmt) | Histamin-Gehalt mittels HPLC bestimmt |
|---|---|---|
| | mg/L | mg/L |
| Rotwein D. O Almansa | 2,63 | 3,10 |
| Rotwein D.O. Rioja | 5,21 | 6,00 |
| Rotwein D. O. Ribera del Duero | 6,27 | 6,80 |
| Rotwein D. O . Vinos de Madrid | 3,93 | 5,10 |
| Rotwein D. O. Ribera del Duero | 3,23 | 4,80 |
| Rotwein D. O. Ribera del Duero | 3,28 | 3,60 |
| Rotwein Vino de Tierra de Castilla | <1.4 | 3,60 |
| Rotwein Vino de Tierra de Castilla | <1.4 | 1,00 |
| Rotwein D. O . Vinos de Madrid | 5,31 | 5,40 |
| Rotwein D. O. Navarra | 5,11 | 5,90 |
| Rotwein D. O. Navarra | <1.4 | <0,5 |
| Rotwein D. O. Navarra | <1.4 | <0,5 |
| Rotwein D. O. Penedés | 7,80 | 8,90 |
| Rotwein D. O . Vinos de Madrid | 1,97 | 1,90 |
| Rotwein D. O . Vinos de Madrid | 1,75 | 1,40 |
| Rotwein D.O. Rioja | 5,17 | 5,40 |
| Rotwein D.O. Rioja | 6,03 | 6,60 |
| Rotwein NRL PT 2013 | 4,97 | 5,23 |
| Rotwein NRL PT 2014 | 9,86 | 10,26 |
| Rotwein D. O. Navarra spike 5 mg/L | 4,33 | 5,30 |
| Rotwein D. O. Navarra spike 10 mg/L | 9,11 | 10,30 |
| Rotwein D. O. Navarra spike 5 mg/L | 4,18 | 5,50 |
| Rotwein D. O. Navarra spike 10 mg/L | 9,11 | 10,20 |
| Rotwein Rioja | 8,59 | 10,20 |
| Rotwein Rioja | 6,70 | 6,70 |
| Rotwein Castilla | 3,60 | 3,60 |
| Rotwein Chianti | 2,09 | 2,80 |
| Rotwein California | 1,14 | <0,5 |

Aus Voruntersuchungen wurde deutlich, dass das Volumen des Tris-Puffers (0.325 M Tris, pH 9.0) kritisch sein kann. Das Volumen von LaNO₃ (0.1 M) und des Na-Phosphatpuffers (0.5 M, pH 7.0) sind unproblematisch. Daher wurde das Volumen des Tris-Puffers (0.325 M, pH 9.0) +/-20% variiert und die Auswirkungen auf einen dotierten Rotwein und einen dotierten Weißwein (Sorte: Weißherbst) untersucht. Bei Erhöhung des Volumens des Tris-Puffers im Rotwein wurde eine Zunahme der Histamin-Konzentration beobachtet. Im Gegensatz dazu wurde beim Weißherbst dieser Effekt genau umgekehrt beobachtet (Tabelle 2). Daraus konnte geschlussfolgert werden, dass 200 µL Tris-Puffer als ausreichend robust angesehen werden können.

**Tabelle 2: Einfluss der Variation des Volumens von Tris-Puffer (0,325 M, pH 9.0) auf das Histamin-Messergebnis; LaNO3-Lösung ist 0.1 M, Phosphatpuffer 0.5 M (pH 7.0)**

| | Tris-Puffer µL | LaNO₃ µL | Phosphatpuffer µL | Histamin-Gehalt mg/L |
|---|---|---|---|---|
| Dornfelder | 240 | 200 | 100 | 11,65 |
| Rotwein | 220 | 200 | 100 | 10,66 |
| Dotiert auf 10 mg/L | *200* | *200* | *100* | *10,62* |
| | 180 | 200 | 100 | 10,26 |
| | 160 | 200 | 100 | 10,16 |
| Spätburgunder | 240 | 200 | 100 | 10,88 |
| Weißherbst | 220 | 200 | 100 | 11,01 |
| Dotiert auf 10 mg/L | *200* | *200* | *100* | *11,23* |
| SO₂ > 260 mg/L | 180 | 200 | 100 | 11,33 |
| | 160 | 200 | 100 | 11,83 |

Wie bereits dargestellt, ist das erfindungsgemäße Verfahren beispielsweise auf die Aufarbeitung von Weiß-, Rose- und Rotweinen mit anschließender enzymatischer Bestimmung der Histamin-Konzentration anwendbar. Die berechnete LoD *(Limit of Detection)* beträgt dabei 0.54 mg/L. Proben in diesem Bereich weisen eine ausreichende Präzision (VK<20%) auf. Die berechnete LoQ (*Limit of Quantification*) (1.44 mg/L) wurde durch Dotierexperimente bestätigt: Es konnte eine Wiederfindung von 95% bei einer relativen Standardabweichung von 3.5% erhalten werden. Die Reproduzierbarkeits-Standardabweichung liegt konzentrationsunabhängig um 0.12 mg/L. Durch die Analyse von 20 Weinproben konnte ein 95%-Vertrauensinterval von 84 - 114 % Wiederfindung berechnet werden.

Stabilitätstestungen belegen eine sehr hohe Stabilität und Unempfindlichkeit der Entstörungs- und Fällungsreagenzien gegen Transport und Stressbedingungen wie hohe oder niedrige Temperaturen. Bisherige Untersuchungen (Tabelle 3) zeigen eine Haltbarkeit gegenüber Temperaturen von 4°C bzw. 45°C von 27 Wochen (etwa 7 Monate).

**Tabelle 3: Vermessung eines Blankrotweines bzw. eines dotierten Rotweines (10 mg/L) in drei verschiedenen Lots der Entstörungsreagenzien nach Lagerung der Reagenzien bei 4 °C, Raumtemperatur (RT, Referenz) und 45°C.**

| **Lot** | Woche | **4°C** | | **RT** | | **45°C** | |
|---|---|---|---|---|---|---|---|
| | | blank mg/L | + 10 mg/L mg/L | blank mg/L | + 10 mg/L mg/L | blank mg/L | + 10 mg/L mg/L |
| 1 | **27** | 0,72 | 10,2 | 0,75 | 10,4 | 0,82 | 10,3 |
| | | 0,72 | 10,5 | 0,68 | 10,1 | 0,82 | 10,3 |
| 2 | **27** | 0,61 | 10,3 | 0,72 | 10,1 | 0,68 | 10,4 |
| | | 0,61 | 10,0 | 0,61 | 10,2 | 0,72 | 10,2 |
| 3 | **27** | 0,72 | 10,3 | 0,75 | 10,5 | 0,82 | 10,4 |
| | | 0,64 | 10,1 | 0,68 | 10,1 | 0,82 | 10,4 |

Unterwirft man die Reagenzien einer simulierten Transportstabilität (6 h leicht auf dem Horizontalschüttler zu Beginn 400/min danach um 150/min; anschließend 17 h bei 4°C; erneut 6 h Horizontalschüttler bei 150/min; 17 h bei 45°C lagern; danach 31 h bei 4°C; weitere längerfristige Lagerung bei Raumtemperatur) so erweisen sich die Reagenzien 6 Monate nach dieser simulierten Transportstabilität als stabil (Tabelle 4).

**Tabelle 4: Vermessung eines Blank-Rotweines bzw. eines dotierten Rotweines (10 mg/L) in drei verschiedenen Lots der Entstörungsreagenzien 6 Monate nach Durchführung einer simulierten Transportstabilität.**

| Lot | Blank mg/L | + 10 mg/L mg/L |
|---|---|---|
| 1 | 0,97 | 11,4 |
| | 0,94 | 11,19 |
| 2 | 0,8 | 10,49 |
| | 0,83 | 10,46 |
| 3 | 0,94 | 10,56 |
| | 0,83 | 10,42 |

Werden die Reagenzien zweimal nacheinander über Nacht bei -20°C eingefroren, erweisen sie sich nach einer Lagerung über 6 Monate bei Raumtemperatur ebenfalls als stabil (Tabelle 5).

**Tabelle 5: Vermessung eines Blankrotweines bzw. eines dotierten Rotweines (10 mg/L) in drei verschiedenen Lots der Entstörungsreagenzien 6 Monate nach zweimaligem Einfrieren bei -20°C.**

| Lot | Blank mg/L | + 10 mg/L mg/L |
|---|---|---|
| 1 | 0,94 | 11,05 |
| | 0,94 | 10,91 |
| 2 | 0,80 | 10,98 |
| | 0,80 | 10,42 |
| 3 | 0,90 | 11,05 |
| | 0,87 | 11,16 |

Extrahierte Proben können bis zu 2 Tage bei 4°C bzw. 1 Tag bei 20-25 °C gelagert werden. Extrakte von Weinen mit Histamin-Konzentrationen außerhalb des Kalibrierungsbereiches können mit Wasser verdünnt werden. Das System reagiert robust gegen eine Variation der Inkubationszeiten oder ein Auslassen des Mischens mittels Vortexer. Das erfindungsgemäße Verfahren zeichnet sich durch eine relative Standardabweichung von kleiner als 4 % für Konzentrationen oberhalb der LoQ aus. Die Methode kann somit bei korrekter Durchführung als hochgradig präzise gelten. Werden andere Lanthaniden anstelle des verwendeten La (III) eingesetzt, so werden unter geringfügiger Anpassung der Pufferstärke in allen Fällen hervorragende Wiederfindungen erzielt (Tabelle 6).

**Tabelle 6: Untersuchung anderer Lanthaniden auf Einsatzfähigkeit zur Entfernung von Störsubstanzen**

| Lanthanid (Wertigkeit) | Lanthanid (mol/L) | Tris pH 9.0 mol/L | Histamin | | |
|---|---|---|---|---|---|
| | | | Rotwein, blank (mg/L) | Rotwein, +10 mg/L (mg(L) | Wiederfindung (%) |
| Ce (III) | 0,1 | 0,325 | < LoQ | 10,21 | 102 |
| Pr (III) | 0,1 | 0,325 | < LoQ | 9,92 | 99 |
| Nd (III) | 0,1 | 0,325 | < LoQ | 9,72 | 97 |
| Sm (III) | 0,1 | 0,3 | < LoQ | 9,61 | 96 |
| Gd (III) | 0,1 | 0,3 | < LoQ | 9,46 | 95 |
| Dy (III) | 0,1 | 0,3 | < LoQ | 9,36 | 94 |
| Er (III) | 0,1 | 0,3 | < LoQ | 9,94 | 99 |
| Yb (III) | 0,1 | 0,3 | < LoQ | 9,23 | 92 |

Die Erfindung ist nicht nur im Speziellen dazu geeignet Störsubstanzen in Wein zu entfernen und im Anschluss eine enzymatische Histamin-Bestimmung durchzuführen. Sie hat darüber hinaus das überaus wichtige Potenzial, Rotweine zu entfärben und so diese Matrix einfach und schnell anderen Analysemethoden wie HPLC oder Lateral Flow Bestimmungen zur Analyse bereitzustellen. Bei anderen problematischen Matrices wie z. B. Urin, Leber und Blut besteht ebenfalls die Möglichkeit, wirkungsvoller und einfacher als zurzeit eine Probenvorbereitung für eine große Anzahl an verschiedenen Analysemethoden zur Verfügung zu stellen.

Die Erfindung wird nachfolgend mit Ausführungsbeispielen näher erläutert.

### Ausführungsbeispiele

### Beispiel 1:

### Fällung von Polyphenolen und Anthocvanen aus Weinproben

1. 200 µL Wein im 2 mL Eppendorfcup vorlegen; Pipettenspitze vorspülen
2. 200 µL Reagenz 1 (0,325 M Tris, pH 9.0) zufügen
3. 200 µL Reagenz 2 (0.1 M LaNO₃ in Wasser) zufügen, vortexen und anschließend 5 min bei RT inkubieren
4. 2 min bei 14000 g und Raumtemperatur zentrifugieren
5. 500 µL Überstand in neues Eppendorfcup transferieren
6. 100 µL Reagenz 3 (0.5 M Phosphatpuffer, pH 7.0) zufügen, vortexen und anschließend 5 min bei RT inkubieren
7. 2 min bei 14000 g und Raumtemperatur zentrifugieren
8. 100 µL vom klaren Überstand im enzymatischen Test in n=2 einsetzen Bedingt durch die Aufarbeitung der Weinprobe muss für die spätere Berechnung der Histamin Konzentration ein Verdünnungsfaktor von 3,6 berücksichtigt werden.

### Beispiel 2:

Die unter Beispiel 1 aufgeführten Volumina können unter Bewahrung der Verhältnisse zueinander beliebig variiert werden. Dabei kann auch die Zentrifugationsgeschwindigkeit verändert werden. So ist auch nachfolgender Ansatz funktional.
1. 2 mL Wein im 10 mL Kunststoff-Röhrchen vorlegen; Pipettenspitze vorspülen
2. 2 mL Reagenz 1 (0,325 M Tris, pH 9.0) zufügen
3. 2 mL Reagenz 2 (0.1 M La(NO₃)₃ in Wasser) zufügen, vortexen und anschließend 5 min bei RT inkubieren
4. 2 min bei 4000 g und Raumtemperatur zentrifugieren
5. 5 mL Überstand in neues Eppendorfcup transferieren
6. 1 mL Reagenz 3 (0.5 M Phosphatpuffer, pH 7.0) zufügen, vortexen und anschließend 5 min bei RT inkubieren
7. 2 min bei 4000 g und Raumtemperatur zentrifugieren
8. 100 µL vom klaren Überstand im enzymatischen Test in n=2 einsetzen

### Beispiel 3:

Bestimmung der Histamin-Konzentration unter Verwendung eines handelsüblichen Testkits z. B. RIDASCREEN® Histamin (enzymatic) der Firma R-Biopharm (Katalog Nr. R1605).

Alle für die enzymatische Bestimmung benötigten Reagenzien auf Raumtemperatur (20 - 25 °C) erwärmen und vor Gebrauch vorsichtig mischen.
1. Eine Mikrotiterplatte wurde mit einem handelsüblichen Elektronenüberträger (20 mg/L) und einem geeigneten Substrat (250 mg/L), welches bei Elektronenübertragung die Farbe wechselt, beschichtet
2. Je 150 µl Reagenz 1 (0.05 M Trispuffer, pH 9) in die entsprechenden Kavitäten der Mikrotiterplatte pipettieren und die Platte vorsichtig manuell schütteln
3. Je 100 µl der Standardlösungen, der Kontrollen bzw. der vorbereiteten Proben aus Beispiel 1 in Doppelbestimmung pipettieren. Die Pipettenspitzen sind vorzuspülen. Anschließend die Platte vorsichtig manuell schütteln.
4. Nach 3 min Inkubation bei Raumtemperatur die Extinktion A1 bei 450 nm ohne Referenzwellenlängefilter messen.
   Wird innerhalb dieser Zeit eine zunehmende Gelbfärbung im Well beobachtet, so sollte auf Ascorbinsäuregehalte im Wein kleiner 250 mg/L getestet werden. Liegt ein säurebetonter Weißwein ohne Ascorbinsäure vor, sollte die Inkubationszeit auf 10 min erhöht werden.
5. 10 µl einer handelsüblichen Histamindehydrogenase (20 U/mL) in jede Kavität pipettieren. Anschließend die Platte vorsichtig manuell schütteln.
6. Nach 10 min Inkubation bei Raumtemperatur die Extinktion A2 bei 450 nm ohne Referenzwellenlängefilter messen.
7. Unter Berücksichtigung des Verdünnungsfaktors die Konzentrationen der Proben berechnen.

### Beispiel 4:

### Untersuchung des Histamingehalts gängiger Weinproben

Es wurden 20 unterschiedliche Weinproben im Handel besorgt, entsprechend dem Ausführungsbeispiel 1 aufgearbeitet und gemäß dem Ausführungsbeispiel 3 die Histamin-Konzentrationen enzymatisch bestimmt. Fast alle Weine wiesen Konzentrationen kleiner 5 mg/L auf. Kursiv markierte Konzentrationen wurden extrapoliert (kleiner LoQ-Wert). Lediglich ein relativ lang gelagerter portugiesischer Rotwein zeigt Gehalte um 10 mg/L (Tabelle 7).

**Tabelle 7: Histamin-Gehalt von handelsüblichen Weinen**

| **Land** | **Region** | **Jahrgang** | **Sorte** | **Typ** | **mg/L** |
|---|---|---|---|---|---|
| DE | Rheinhessen | 2013 | Müller-Thurgau | weiß | *0,5* |
| HU | Felsö-Magyarorszag | 2013 | Grüner Veltliner | weiß | *0,4* |
| DE | Rheingau | 2014 | Riesling | weiß | *1,2* |
| IT | Trentino | 2013 | Cuvee | rosé | *0,3* |
| IT | Bardolino | 2013 | Cuvee | rose | *0,4* |
| DE | Rheingau | 2013 | Pinot noir | rose | *0,4* |
| DE | Rheingau | 2014 | Pinot Noir | rot | *0,0* |
| DE | Rheingau | 2012 | Cuvee | rot | *0,2* |
| DE | Rheinhessen | 2011 | Pinot Noir | rot | *0,2* |
| IT | Piemont | 2013 | Barbera D'asti | rot | *0,4* |
| DE | Rheinhessen | 2014 | Dornfelder | rot | *0,5* |
| ES | Rioja | 2006 | Cuvee | rot | *0,6* |
| FR | Bordeaux | 2013 | Cuvee | rot | *0,6* |
| FR | Cotes Du Rhone | 2013 | Grenache-Syrah | rot | *0,9* |
| PT | Alentejano | 2009 | Cuvee | rot | 2,2 |
| IT | Sicily | 2013 | Nero D'Avola | rot | 2,4 |
| IT | Sicily | 2013 | Nero D'Avola-Merlot | rot | 4,1 |
| ES | Ribera de Duero | 2011 | Tempranillo | rot | 4,5 |
| IT | Umbria | 2010 | Cuvee | rot | 4,8 |
| IT | Trentino | 2012 | Merlot | rot | 5,5 |
| PT | Duoro | 2009 | Cuvee | rot | 10,7 |

### Beispiel 5:

### Verdünnbarkeit des Extraktes einer hochkonzentrierten Weinprobe

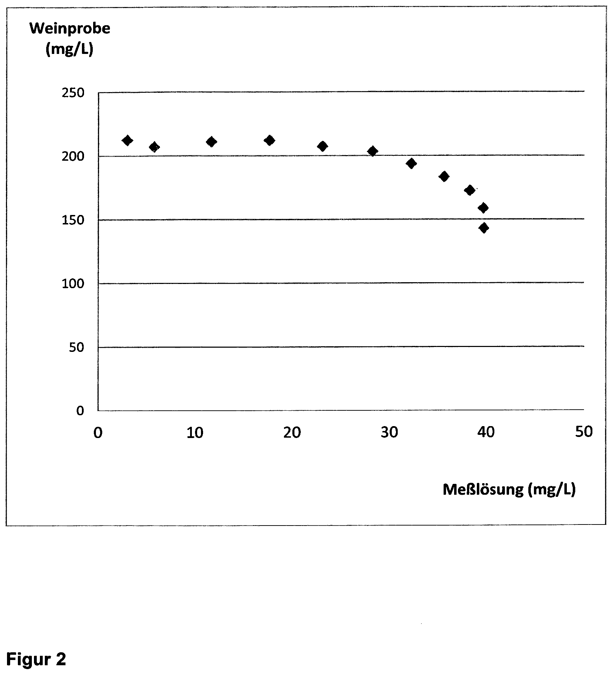

Eine Dornfelder Rotweinprobe wird auf 200 mg/L Histamin dotiert und aufgearbeitet. Im Anschluss wird dieser Extrakt mit Wasser verdünnt, um den gesamten Messbereich abzudecken. Diese Messlösungen werden im enzymatischen Assay vermessen. Das enzymatische System wurde für andere Validierungen auf Linearität überprüft. Diese Überprüfung ergab eine Linearität bis etwa 25 mg/L in der Messlösung. Beim Vergleich mit Daten in Tabelle 6 kann dieser Linearitätsanspruch aufrechterhalten werden. Ab etwa 30 mg/L Histamin in der Messlösung muss mit signifikant niedrigeren Konzentrationen gerechnet werden. Eine graphische Auswertung der Daten aus Tabelle 8 wird in Figur 2 gegeben. Im validierten Messbereich von etwa 1 bis 20 mg/L liegt somit eine Verdünnbarkeit der Extrakte vor.

**Tabelle 8: Verdünnbarkeit der Messlösung**

| Wein mg/L | Messlösung mg/L | Faktor Verdünnung | Wein mg/L |
|---|---|---|---|
| 200 | *39,8* | 3,6 | *143,1* |
| | *39,7* | 4 | *158,8* |
| | *38,3* | 4,5 | *172,4* |
| | *35,7* | 5,14 | *183,3* |
| | *32,3* | 6 | *193,6* |
| | *28,2* | 7,2 | *203,3* |
| | 23,1 | 9 | 207,5 |
| | 17,7 | 12 | 212,2 |
| | 11,7 | 18 | 211,0 |
| | 5,75 | 36 | 207,0 |
| | 2,95 | 72 | 212,4 |

### Beispiel 6:

### Einfluss unterschiedlicher Inkubationszeiten

Untersucht wurde der Einfluss unterschiedlicher Inkubationszeiten in der Probenaufarbeitung. Es wurden die Inkubationszeiten aus Beispiel 1 nach Zugabe des Tris-Puffers (Inkubation 1) und nach Zugabe des Na-Phosphatpuffers (Inkubation 2) variiert und der Einfluss auf den anschließend bestimmten Histamin-Gehalt untersucht (Tabelle 9).

**Tabelle 9: Einfluss der Inkubationszeiten auf das Histamin-Messergebnis einer dotierten Rotweinprobe (Dornfelder)**

| Inkubation 1 min | Inkubation 2 min | Gehalt mg/L |
|---|---|---|
| 4 | 4 | 10,75 |
| 4 | 5 | 10,57 |
| **4** | **20** | 10,49 |
| 5 | 4 | 11,91 |
| *5* | *5* | *10,68* |
| 5 | 20 | 10,64 |
| **20** | **4** | 11,54 |
| 20 | 5 | 10,49 |
| 20 | 20 | 10,42 |

Die Ergebnisse in Tabelle 9 belegen eindeutig, dass kein Effekt beobachtbar ist. Die Inkubationszeiten können als robust angesehen werden und sind mit jeweils 5 min als ausreichend anzusehen.

### Beispiel 7:

Bestimmung der Clenbuterol-Konzentration unter Verwendung eines handelsüblichen Testkits z. B. RIDASCREEN® Clenbuterol der Firma R-Biopharm (Katalog Nr. R1711).

Alle für die enzymatische Bestimmung benötigten Reagenzien auf Raumtemperatur (20 - 25 °C) erwärmen und vor Gebrauch vorsichtig mischen.
1. Eine Mikrotiterplatte wurde mit spezifischen Antikörpern gerichtet gegen Clenbuterol beschichtet (Die Beschichtungsmenge ist abhängig von Antikörper-Lot und den Eigenschaften des verwendeten Clenbuterol-Enzym-Konjugates, liegt aber meist im Bereich von 1 µg pro Vertiefung der Mikrotiterplatte)
2. 50 µL Standardlösung bzw. extrahierte Probe in die Vertiefungen der Mikrotiterplatte pipettieren
3. 30 min bei Raumtemperatur im Dunkeln inkubieren und anschließend mit je 250 µL PBS/Tween-Puffer dreimal sorgfältig waschen; zwischen jedem Waschschritt und am Ende auf saugfähigen Labortüchern kräftig ausklopfen
4. Je 50 µL Clenbuterol-Enzym-Konjugat in die Vertiefungen pipettieren (Konjugat-Menge ist chargenspezisch und abhängig von der Antikörper-Menge pro Vertiefung)
5. 15 min bei Raumtemperatur im Dunkeln inkubieren und anschließend mit je 250 µL PBS/Tween-Puffer dreimal sorgfältig waschen; zwischen jedem Waschschritt und am Ende auf saugfähigen Labortüchern kräftig ausklopfen
6. Je 100 µL Substratlösung (Tetramethylbenzidin/H₂O₂ in den üblichen Konzentrationen) in die Vertiefungen pipettieren, 15 min bei Raumtemperatur in Dunkeln inkubieren und anschließend mit 100 µL 1 N H₂SO₄ die Reaktion abstoppen
7. Die Messung erfolgt in Mikrotiterplattenphotometer bei 450 nm
8. Die Konzentrationsbestimmung der Proben erfolgt unter Verwendung der Werte der Standardkurve; die Verdünnungsfaktoren bedingt durch die Extraktion der Proben vor der Messung muss berücksichtigt werden

### Beispiel 8:

### Fällung von Polyphenolen aus Urinproben

Bedingt durch die hohen Gehalte an Polyphenolen im Futter, zeigen Urinproben von Schwein und Rind beim Einsatz in enzymimmunologischen Systemen Matrixeffekte, die eine schlechte Wiederfindung von Clenbuterol bewirken. Zur Verringerung dieses Effektes wurde vor Bestimmung des Clenbuterolgehaltes folgendermaßen aufgearbeitet:
- 300 µl Urinprobe mit 300 µl 0.1 M La(NO₃)₃ versetzen
- mischen, 5 min bei RT inkubieren, danach 2 min bei 14000 g zentrifugieren
- 300 µl des Überstandes abnehmen und mit 150 µl 0.5 M Phosphatpuffer ersetzen
- mischen, 5 min bei RT inkubieren und 2 min bei 14000 g zentrifugieren

**Tabelle 10: Parallele Messung von 3 Rinderurinen und 4 Schweineurinen nach Aufarbeitung mittels Lathanidenfällung oder direktem Einsatz der Urine nach Verdünnung mit Testpuffer; da die Urine bereits einen optimalen pH-Wert für die Fällung aufweisen, kann auf eine pH-Wert-Einstellung mittels Trispuffer verzichtet werden; als Dotierlevel wurde 0.5 µg/kg gewählt.**

| Urin | mit Fällung WF (%) | ohne Fällung WF (%) |
|---|---|---|
| Rind 2045-20 | 82% | 47% |
| Rind 2240-6 | 104% | 52% |
| Rind 2240-7 | 84% | 55% |
| Schwein 2236-9 | 80% | 66% |
| Schwein 2236-11 | 93% | 56% |
| Schwein FEVal 12 | 100% | 59% |
| Schwein FEVal 18 | 92% | 44% |

Die Ergebnisse in Tabelle 10 belegen die hervorragende Eignung der Fällung zur Probenvorbereitung von Urin in der Clenbuterol-Messung mittels ELISA.

### Beispiel 9:

### Fällung von Polyphenol-verwandten Stoffklassen aus Milch

Milch weist zwar keine nennenswerten Gehalte an Polyphenolen auf, enthält aber ebenfalls Stoffklassen, die sich durch wasserlösliche Salze der dreiwertigen Lanthaniden durch Fällung entfernen lassen. Ohne diese Entfernung kommt es im nachgeschalteten enzymimmunologischen System zu schlechten Wiederfindungen. Zur Beseitigung dieses Effektes wurde vor Bestimmung des Clenbuterolgehaltes folgendermaßen aufgearbeitet:
- 300 µl Milchprobe mit 300 µl 0.1 M La(NO₃)₃ versetzen
- mischen, 5 min bei RT inkubieren, danach 2 min bei 14000 g zentrifugieren
- 300 µl des Überstandes abnehmen und mit 150 µl 0.5 M Phosphatpuffer ersetzen
- mischen, 5 min bei RT inkubieren und 2 min bei 14000 g zentrifugieren

**Tabelle 11: Parallele Messung von 6 verschiedenen Milchproben nach Aufarbeitung mittels Lathanidenfällung oder direktem Einsatz der Milch nach Verdünnung mit Testpuffer; da die Milchproben bereits einen optimalen pH-Wert für die Fällung aufweisen, kann auf eine pH-Wert-Einstellung mittels Trispuffer verzichtet werden; als Dotierlevel wurde 0.5 µg/L gewählt.**

| Milch | mit Fällung WF (%) | ohne Fällung WF (%) |
|---|---|---|
| 31.08.2015 | 85% | 72% |
| 01.09.2015 | 84% | 62% |
| FEVal 1 | 111% | 69% |
| FEVal 2 | 100% | 81% |
| FEVal 11 | 82% | 73% |
| FEVal 12 | 84% | 65% |

Die Ergebnisse in Tabelle 11 belegen die hervorragende Eignung der Fällung zur Probenvorbereitung von Milch in der Clenbuterol-Messung mittels ELISA.

### Beispiel 10:

### Fällung von Polyphenolen aus Futtermitteln

Rinder- und Schweinefuttermittel enthalten hohe Mengen an Polyphenolen und zeigen nach einfacher Lösungsmittelextraktion in enzymimmunologischen Systemen Matrixeffekte, die ein falsch-positives Ergebnis vortäuschen und dadurch bedingt die Nachweisgrenze der verwendeten analytischen Methode erhöhen. Zur Verringerung dieses Effektes wurde vor Bestimmung des Clenbuterolgehaltes folgendermaßen aufgearbeitet:
- 2 g Futtermittel mit 5 ml 50 % Acetonitril versetzen und 20 min schütteln
- 10 min auf Eis inkubieren und anschließend bei 14000 g 2 min zentrifugieren
- 150 µL der klaren Überstandes mit 300 µL 0.325 M Trispuffer und 200 µL 0.1 M La(NO₃)₃-Lösung versetzen
- 5 min bei Raumtemperatur inkubieren; anschließend bei 14000 g 2 min zentrifugieren
- 100 µl 0.5 M Phosphatpuffer zusetzen, mischen, 5 min bei Raumtemperatur inkubieren und anschließend bei 14000 g 2 min zentrifugieren

Die Standardaufarbeitung mittels Lösungsmittelextraktion ist wie folgt:
- 1 g Futtermittel mit 3 mL Acetonitril versetzen und 20 min schütteln
- 2 mL des Überstandes evaporieren und in 3 mL n-Hexan resuspendieren
- 1 mL Waschpuffer (des verwendeteten ELISA) zufügen
- 20 min schütteln und anschließend die untere wässrige Phase im Test einsetzen

**Tabelle 12: Parallele Messung von verschiedenen Futtermitteln für Schwein und Rind nach Aufarbeitung mittels Lathanidenfällung oder Extraktion der Futtermittel mithilfe einer Acetonitril-Extraktion; als Dotierlevel wurde 0.85 µg/kg gewählt.**

| Futtermittel | Ohne Fällung WF (%) | Mit Fällung WF (%) |
|---|---|---|
| Schwein (Mast) | 12% | 104% |
| Rind (Milch) | 42% | 96% |
| Kalb | 21% | 83% |
| Schwein (Sauen) | 17% | 84% |
| Rind (Mast) | 28% | 95% |

Die Ergebnisse in Tabelle 12 belegen die hervorragende Eignung der Fällung zur Probenvorbereitung von Futtermitteln in der Clenbuterol-Messung mittels ELISA.

### Beispiel 11:

### Fällung von Polyphenol-verwandten Stoffklassen aus Leber

Leber von Schweinen und Rindern weisen zwar keine nennenswerten Gehalte an Polyphenolen auf, enthalten aber offensichtlich ebenfalls Stoffklassen, die sich durch wasserlösliche Salze der dreiwertigen Lanthaniden durch Fällung entfernen lassen. Ohne diese Entfernung kommt es im nachgeschalteten enzymimmunologischen System zu schlechten Wiederfindungen. Zur Beseitigung dieses Effektes wurde vor Bestimmung des Clenbuterolgehaltes folgendermaßen aufgearbeitet:
- 2 g Leber mit 3 ml 80 % Acetonitril versetzen und schütteln
- 15 min auf Eis inkubieren und anschließend bei 10 °C 10 min zentrifugieren
- 100 µL des Überstandes mit 300 µL 0.08 M Trispuffer pH 9.0 versetzen
- Zugabe von 100 µl 0.1 M La(NO₃)₃
- mischen, 5 min bei RT inkubieren und 2 min bei 14000 g zentrifugieren
- 400 µl Überstand abnehmen und 50 µL 0.5 M Phosphatpuffer zusetzen
- mischen, 5 min bei RT inkubieren und 2 min bei 14000 g zentrifugieren

Die Standardaufarbeitung mittels Lösungsmittelextraktion ist wie folgt:
- 3 g Leber mit 8 mL Acetonitril und 1 mL Ethylacetat versetzen
- 30 min schütteln und anschließend bei 4000 g 10 min zentrifugieren
- 4 ml Überstand evaporieren und in 2 mL Testpuffer aufnehmen

**Tabelle 13: Parallele Messung von Leber (Schwein und Rind) nach Aufarbeitung mittels Lathanidenfällung oder Extraktion der Lebern mithilfe eines Acetonitril-Ethylacetatgemisches; als Dotierlevel wurde 5 µg/kg gewählt.**

| Leber | mit Fällung WF (%) | ohne Fällung WF (%) |
|---|---|---|
| RL14-#1 | 79% | 26% |
| 2312-2 | 100% | 42% |
| 2312-4 | 105% | 31% |
| SL14-#4 | 76% | 36% |

Die Ergebnisse in Tabelle 13 belegen die hervorragende Eignung der Fällung zur Probenvorbereitung von Leber in der Clenbuterol-Messung mittels ELISA.

### Beispiel 12:

### Fällung von Polyphenol-verwandten Stoffklassen aus Blut

Wegen der hohen Wirksamkeit von Lanthanid-haltigen Fällungsreagenzien in Milch (Beispiel 8) und Leber (Beispiel 9) kann gefolgert werden, dass es beim Einsatz von Blut ebenfalls zu einer Fällung von Störsubstanzen kommen kann und dieser Effekt analytisch ausgenutzt werden kann.

### Beispiel 13:

### Fällung von Polyphenolen und Anthocyanen mit Hilfe von Cerium (III)

1. 200 µL Wein im 2 mL Eppendorfcup vorlegen
2. 200 µL Reagenz 1 (0,325 M Tris, pH 9.0) zufügen
3. 200 µL Reagenz 2 (0.1 M Ce(NO₃)₃ in Wasser) zufügen, vortexen und anschließend 5 min bei RT inkubieren
4. 2 min bei 14000 g und Raumtemperatur zentrifugieren
5. 500 µL Überstand in neues Eppendorfcup transferieren
6. 100 µL Reagenz 3 (0.5 M Phosphatpuffer, pH 7.0) zufügen, vortexen und anschließend 5 min bei RT inkubieren
7. 2 min bei 14000 g und Raumtemperatur zentrifugieren
8. 100 µL vom klaren Überstand im enzymatischen Test in n=2 einsetzen

### Beispiel 14:

### Fällung von Polyphenolen und Anthocyanen mit Hilfe von Praseodym (III)

1. 200 µL Wein im 2 mL Eppendorfcup vorlegen
2. 200 µL Reagenz 1 (0,325 M Tris, pH 9.0) zufügen
3. 200 µL Reagenz 2 (0.1 M Pr(NO₃)₃ in Wasser) zufügen, vortexen und anschließend 5 min bei RT inkubieren
4. 2 min bei 14000 g und Raumtemperatur zentrifugieren
5. 500 µL Überstand in neues Eppendorfcup transferieren
6. 100 µL Reagenz 3 (0.5 M Phosphatpuffer, pH 7.0) zufügen, vortexen und anschließend 5 min bei RT inkubieren
7. 2 min bei 14000 g und Raumtemperatur zentrifugieren
8. 100 µL vom klaren Überstand im enzymatischen Test in n=2 einsetzen

### Beispiel 15:

### Fällung von Polyphenolen und Anthocyanen mit Hilfe von Neodym (III)

1. 200 µL Wein im 2 mL Eppendorfcup vorlegen
2. 200 µL Reagenz 1 (0,325 M Tris, pH 9.0) zufügen
3. 200 µL Reagenz 2 (0.1 M Nd(NO₃)₃ in Wasser) zufügen, vortexen und anschließend 5 min bei RT inkubieren
4. 2 min bei 14000 g und Raumtemperatur zentrifugieren
5. 500 µL Überstand in neues Eppendorfcup transferieren
6. 100 µL Reagenz 3 (0.5 M Phosphatpuffer, pH 7.0) zufügen, vortexen und anschließend 5 min bei RT inkubieren
7. 2 min bei 14000 g und Raumtemperatur zentrifugieren
8. 100 µL vom klaren Überstand im enzymatischen Test in n=2 einsetzen

### Beispiel 16:

### Fällung von Polyphenolen und Anthocyanen mit Hilfe von Samarium (III)

9. 200 µL Wein im 2 mL Eppendorfcup vorlegen
10.200 µL Reagenz 1 (0,3 M Tris, pH 9.0) zufügen
11.200 µL Reagenz 2 (0.1 M Sm(NO₃)₃ in Wasser) zufügen, vortexen und anschließend 5 min bei RT inkubieren
12.2 min bei 14000 g und Raumtemperatur zentrifugieren
13.500 µL Überstand in neues Eppendorfcup transferieren
14.100 µL Reagenz 3 (0.5 M Phosphatpuffer, pH 7.0) zufügen, vortexen und anschließend 5 min bei RT inkubieren
15.2 min bei 14000 g und Raumtemperatur zentrifugieren
16.100 µL vom klaren Überstand im enzymatischen Test in n=2 einsetzen

### Beispiel 17:

### Fällung von Polyphenolen und Anthocyanen mit Hilfe von Gadolinium (III)

1. 200 µL Wein im 2 mL Eppendorfcup vorlegen
2. 200 µL Reagenz 1 (0,3 M Tris, pH 9.0) zufügen
3. 200 µL Reagenz 2 (0.1 M Gd(NO₃)₃ in Wasser) zufügen, vortexen und anschließend 5 min bei RT inkubieren
4. 2 min bei 14000 g und Raumtemperatur zentrifugieren
5. 500 µL Überstand in neues Eppendorfcup transferieren
6. 100 µL Reagenz 3 (0.5 M Phosphatpuffer, pH 7.0) zufügen, vortexen und anschließend 5 min bei RT inkubieren
7. 2 min bei 14000 g und Raumtemperatur zentrifugieren
8. 100 µL vom klaren Überstand im enzymatischen Test in n=2 einsetzen

### Beispiel 18:

### Fällung von Polyphenolen und Anthocvanen mit Hilfe von Dysprosium (III)

9. 200 µL Wein im 2 mL Eppendorfcup vorlegen
10.200 µL Reagenz 1 (0,3 M Tris, pH 9.0) zufügen
11.200 µL Reagenz 2 (0.1 M Dy(NO₃)₃ in Wasser) zufügen, vortexen und anschließend 5 min bei RT inkubieren
12.2 min bei 14000 g und Raumtemperatur zentrifugieren
13.500 µL Überstand in neues Eppendorfcup transferieren
14.100 µL Reagenz 3 (0.5 M Phosphatpuffer, pH 7.0) zufügen, vortexen und anschließend 5 min bei RT inkubieren
15.2 min bei 14000 g und Raumtemperatur zentrifugieren
16.100 µL vom klaren Überstand im enzymatischen Test in n=2 einsetzen

### Beispiel 19:

### Fällung von Polyphenolen und Anthocvanen mit Hilfe von Erbium (III)

17.200 µL Wein im 2 mL Eppendorfcup vorlegen
18.200 µL Reagenz 1 (0,3 M Tris, pH 9.0) zufügen
19.200 µL Reagenz 2 (0.1 M Er(NO₃)₃ in Wasser) zufügen, vortexen und anschließend 5 min bei RT inkubieren
20.2 min bei 14000 g und Raumtemperatur zentrifugieren
21.500 µL Überstand in neues Eppendorfcup transferieren
22.100 µL Reagenz 3 (0.5 M Phosphatpuffer, pH 7.0) zufügen, vortexen und anschließend 5 min bei RT inkubieren
23.2 min bei 14000 g und Raumtemperatur zentrifugieren
24.100 µL vom klaren Überstand im enzymatischen Test in n=2 einsetzen

### Beispiel 20:

### Fällung von Polyphenolen und Anthocyanen mit Hilfe von Ytterbium (III)

1. 200 µL Wein im 2 mL Eppendorfcup vorlegen
2. 200 µL Reagenz 1 (0,3 M Tris, pH 9.0) zufügen
3. 200 µL Reagenz 2 (0.1 M Yb(NO₃)₃ in Wasser) zufügen, vortexen und anschließend 5 min bei RT inkubieren
4. 2 min bei 14000 g und Raumtemperatur zentrifugieren
5. 500 µL Überstand in neues Eppendorfcup transferieren
6. 100 µL Reagenz 3 (0.5 M Phosphatpuffer, pH 7.0) zufügen, vortexen und anschließend 5 min bei RT inkubieren
7. 2 min bei 14000 g und Raumtemperatur zentrifugieren
8. 100 µL vom klaren Überstand im enzymatischen Test in n=2 einsetzen

### Legende zu den Figuren

- Figur 1:: Vergleich zwischen HPLC-Referenzmethodik und dem erfindungsgemäßen Verfahren einer Kombination aus Fällung von Polyphenolen und Anthocyanen und anschließender enzymatischer Bestimmung der Histamin-Konzentration anhand von 23 verschiedenen Rotweinen.
- Figur 2:: Graphische Auswertung der Extrakt-Verdünnbarkeit. Der Kalibrierungsbereich des enzymatischen Assays liegt zwischen 1 und 20 mg/L.

## Patentansprüche

1. Verfahren zur Vorbereitung der Analytik von Proben biologischen Ursprungs, **dadurch gekennzeichnet, dass** die biologische Probe in einem Puffersystem mit pH 7 - 10 aufgenommen wird, anschließend vorhandene Störsubstanzen mit wasserlöslichen Salzen von dreiwertigen Lanthaniden ausgefällt, überschüssige wasserlösliche Salze von dreiwertigen Lanthaniden gefällt und als Niederschlag abtrennt werden und mit dem Überstand die Analytik durchgeführt wird.

2. Verfahren nach Anspruch 1 - 2, **dadurch gekennzeichnet, dass** als dreiwertiges Lanthanid Lanthan(III)nitrat in Lösung eingesetzt wird.

3. Verfahren nach Anspruch 1 - 2, **dadurch gekennzeichnet, dass** nach der Ausfällung der Störsubstanzen die überschüssigen Lanthaniden in schwerlösliche Salze, bevorzugt Sulfate, Oxalate, Carbonate oder Phosphate überführt und abgetrennt werden.

4. Verfahren nach Anspruch 1 - 3, **dadurch gekennzeichnet, dass** als Proben biologischen Ursprungs Lebensmittel, Futtermittel oder Körperbestandteile einsetzt werden.

5. Verfahren nach Anspruch 1 - 4 **dadurch gekennzeichnet, dass** als Proben biologischen Ursprungs Weinproben in einem Puffersystem mit pH 7-10 aufgenommen werden, anschließend vorhandene Polyphenole und/oder Anthocyane mit wasserlöslichen Salzen von dreiwertigen Lanthaniden ausgefällt werden, überschüssige dreiwertige Lanthanide in schwerlösliche Salze überführt und abtrennt wird und mit dem Überstand eine enzymatische Bestimmung des Histamin-Gehalts durchgeführt wird.

6. Verfahren nach Anspruch 1 - 5, **dadurch gekennzeichnet, dass** als Lebensmittel Getränke wie Wein, Säfte, Bier, Teesorten oder Milch, Milchprodukte, Honig, Fleisch oder Fisch einsetzt werden.

7. Verfahren nach Anspruch 1 - 6, **dadurch gekennzeichnet, dass** als Körperbestandteile beispielsweise Blut, Gewebe, Speichel oder Urin eingesetzt werden.

8. Verwendung des Verfahrens gemäß Anspruch 1-7 zur Entfernung von Störsubstanzen aus Proben biologischen Ursprungs zur Vorbereitung auf eine anschließende Analytik

9. Verwendung nach Anspruch 8 zur Entfernung von Polyphenolen und/oder Anthocyanen aus Weinproben.

## Claims

1. A method for preparing the analysis of samples of biological origin, **characterized in that** the biological sample is held in a buffer system with pH 7-10, subsequently, interfering substances present are precipitated out with soluble salts of trivalent lanthanides, excess soluble salts are precipitated out of trivalent lanthanides and separated as precipitate, and the analysis is conducted with the supernatant.

2. The method according to claim 1-2, **characterized in that** the lanthanum(III) nitrate is used in solution as a trivalent lanthanide.

3. The method according to claim 1-2, **characterized in that** following precipitation out of the interfering substances, the excess lanthanides are transferred into salts that are not readily soluble, preferably sulphates, oxalates, carbonates or phosphates, and separated.

4. The method according to claim 1-3, **characterized in that** as samples of biological origin, food, animal feed or body components are used.

5. The method according to claim 1-4, **characterized in that** as samples of biological origin, wine samples are held in a buffer system with pH 7-10, then polyphenols and/or anthocyanins present are precipitated out with water-soluble salts of trivalent lanthanides, excess trivalent lanthanides are transferred into salts that are not readily soluble and separated, and with the supernatant, an enzymatic determination of the histamine content is conducted.

6. The method according to claim 1-5, **characterized in that** as food, drinks such as wine, juices, beer, tea varieties or milk, dairy products, honey, meat or fish are used.

7. The method according to claim 1-6, **characterized in that** as body components, blood, tissue, saliva or urine are used, for example.

8. The use of the method according to claim 1-7 for removing interfering substances from samples of biological origin for preparation for subsequent analysis.

9. The use of claim 8 to remove polyphenols and/or anthocyanins from wine samples.

## Revendications

1. Procédé pour la préparation de l'analyse d'échantillons d'origine biologique, **caractérisé par le fait que** l'échantillon biologique est intégré dans un système tampon avec un pH de 7 à 10, que les substances interférentes présentes sont ensuite précipitées avec des sels solubles dans l'eau de lanthanides trivalents, que l'excédent de sels solubles dans l'eau de lanthanides trivalents est précipité et séparé comme précipité et que l'analyse est réalisée avec le surnageant.

2. Procédé conformément à la revendication n°1 à n°2, **caractérisé par le fait que**, comme lanthanide trivalent, du nitrate de lanthane (III) est employé dans la solution.

3. Procédé conformément à la revendication n°1 à n°2, **caractérisé par le fait que**, après la précipitation des substances interférentes, l'excédent de lanthanides est transféré dans des sels difficilement solubles, de préférence des sulfates, des oxalates, des carbonates ou des phosphates, et séparé.

4. Procédé conformément à la revendication n°1 à n°3, **caractérisé par le fait que**, comme échantillons d'origine biologique, des denrées alimentaires, des aliments pour animaux ou des constituants corporels sont utilisés.

5. Procédé conformément à la revendication n°1 à n°4, **caractérisé par le fait que**, comme échantillons d'origine biologique, des échantillons de vin sont intégrés dans un système tampon avec un pH de 7 à 10, que les polyphénols et/ou anthocyanes présents sont ensuite précipités avec des sels solubles dans l'eau de lanthanides trivalents, que l'excédent de lanthanides trivalents est transféré dans des sels difficilement solubles et séparé et qu'une détermination enzymatique de la teneur en histamine est réalisée avec le surnageant.

6. Procédé conformément à la revendication n°1 à n°5, **caractérisé par le fait que**, comme denrées alimentaires, des boissons comme du vin, du jus de fruits, de la bière, des thés ou du lait, des produits laitiers, du miel, de la viande ou du poisson sont utilisés.

7. Procédé conformément à la revendication n°1 à n°6, **caractérisé par le fait que**, comme constituants corporels, par exemple du sang, des tissus, des muqueuses ou de l'urine sont utilisés.

8. Utilisation du procédé conformément à la revendication n°1 à n°7 pour l'élimination de substances interférentes issues d'échantillons d'origine biologique en vue de la préparation d'une analyse ultérieure.

9. Utilisation conformément à la revendication n°8 pour l'élimination de polyphénols et/ou d'anthocyanes issus d'échantillons de vin.
